# EUROPEAN PATENT SPECIFICATION

(11) **EP 1 363 489 B1**
(45) Date of publication and mention of the grant of the patent: **20.10.2004**
(21) Application number: 02711551.8
(22) Date of filing: 18.02.2002
(51) Int. Cl.: A01L 15/00, A61B 5/103

(54) **METHOD AND SYSTEM FOR MONITORING THE HOOVES OF ANIMALS**
VERFAHREN UND EINRICHTUNG ZUR UBERWACHUNG VON TIERHUFEN
PROCEDE ET SYSTEME DE SURVEILLANCE DE SABOTS D'ANIMAUX

(30) Priority: 19.02.2001 NL 1017390
(43) Date of publication of application: 26.11.2003
(73) Proprietor: Imag B.V., 6700 AA Wageningen (NL)
(72) Inventor: HOGEWERF, Pieter, Hendrik, NL-6932 GH Westervoort (NL); IPEMA, Albertus, Hendrik, NL-6708 NW Wageningen (NL)
(74) Representative: Prins, Adrianus Willem, Mr. Ir.
(86) International application number: PCT/NL2002/000107
(87) International publication number: WO 2002/065833

(56) References cited:
- EP-A- 0 970 657
- DE-U- 9 107 369
- US-A- 5 659 395
- US-A- 5 736 656
- US-A- 6 038 935

## Description

The present invention relates to a method and system for monitoring the hooves of animals. The invention relates in particular to monitoring hoof health by measuring hoof pressure profiles, step distance and step speed of cloven-hoofed animals (for example cattle). Monitoring is carried out in order to be able to detect any abnormalities in hoof health at an early stage and to be able to take measures if necessary.

The principle of detecting a pressure profile is known from European Patent Application EP-A 0 970 657, which describes a detector mat by means of which footprints are digitised. With this installation the detector mat is connected to a recording medium in which the measured data are stored. Relating pressure to a digitised image is effected using a film, the electrical resistance of which is dependent on the pressure that is exerted on the film. With the aid of a matrix of electrical contact points which are arranged under the film, the resistance of the film between the raster points is measured. Via multiplexers, amplifiers and analogue to digital conversion the signals are interfaced with a processing unit (for example a personal computer). The film is a nonlinear force recorder which, moreover, ages as a result of loading. These aspects are compensated for on the one hand by calibrating the detector mat signal with the aid of linear force recorders by means of which the total force that is exerted on the mat by the animal is recorded and, on the other hand, by conscientiously keeping an up-to-date "account" in the database of the loading (wear) on the various parts of the mat.

Document US-5 736 656 discloses a method and apparatus for detecting and analysing a pressure profile of an animal hoof.

The aim of the present method is to record abnormalities in pressure profiles, in foot movements and combinations thereof for (for example cloven-hoofed) animals at an early stage and consequently to take preventive measures in good time so that better hoof health can be achieved.

Said aim is achieved by a method according to Claim 1. Further advantageous embodiments are described in the dependent claims.

Using the method according to the present invention it is possible to monitor the state of health of the animals, related to the hooves of the animals, on the basis of detected animal hoof prints.

In a further aspect of the present invention this aim is achieved by a system according to Claim 13. Further advantageous embodiments are described in the dependent system claims.

Using the present installation it is possible entirely automatically to detect and to store data relating to hoof prints of animals and to analyse these data with regard to possible abnormalities and then to take measures to combat the abnormalities.

The invention will be explained in more detail with the aid of a description of an illustrative embodiment, with reference to the appended drawings, in which:
Fig. 1 shows a block diagram of one embodiment of the system according to the present invention; and
Fig. 2 shows a side view of a possible spatial arrangement of the various elements of the system in Fig. 1.

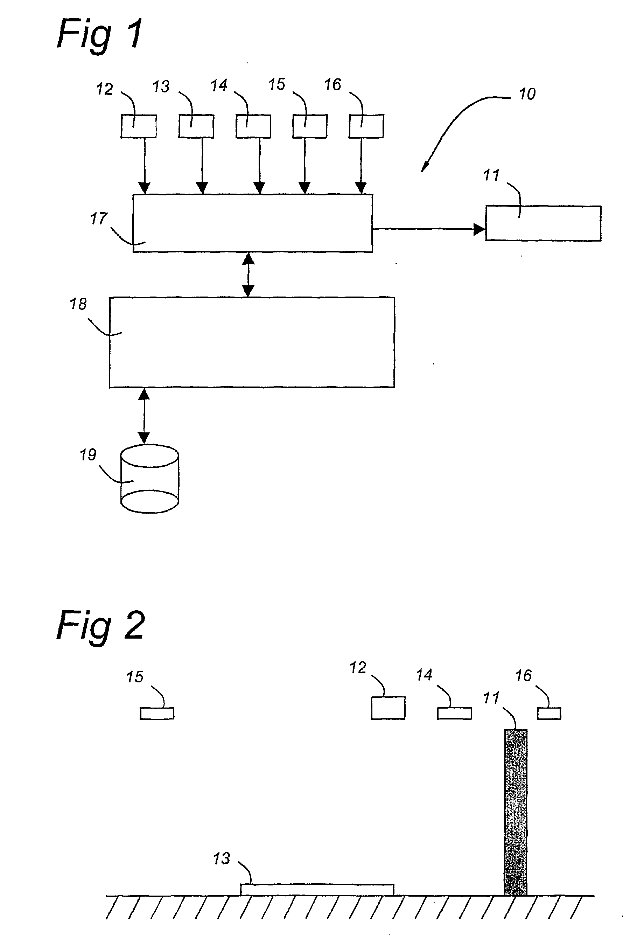

A block diagram of a possible embodiment of the system according to the present invention is shown in Fig. 1. The system 10 comprises processing means 18, such as a PC, and memory means 19, which are connected to the processing means 18. The processing means 18 are furthermore connected via an interface unit 17 to a number of further elements.

The elements consist of detection means 13, such as a mat by means of which the hoof pressure for various surface units can be determined, using, for example, a matrix of pressure recording elements. Furthermore, the system 10 comprises an access system 11, which controls the physical access of an animal to the detection means 13. One or more presence sensors 14, 15 are provided to detect the presence of the animal. Identification means 12, which detect information relating to the identity of the animal and transmit this to the processing means 18, are optionally also present.

The detection means 13 which were developed for human applications can be adapted so that these can be employed for the detection of a three-dimensional animal hoof print. The hoof print referred to here is understood to be the digital reproduction of the various pressures per surface unit which a hoof exerts on a walking or standing surface.

The condition of the hooves of the animals concerned can be derived on the basis of recorded prints. The condition of an animal's hooves is expressed both in static recordings and in dynamic recordings. In the case of the static recordings unbalanced prints in particular are characteristic of various abnormalities. In the case of the dynamic recordings abnormalities in step distances, step speeds and the like can also be observed in addition to the said unbalanced prints. By comparing recent prints with previous prints it is also possible to establish changes and flag these if necessary. The results of the comparisons (abnormalities) can be recorded in a management system and can be part of an integral management system, which, for example, is implemented in the processing means 18.

The present invention is targeted at obtaining reliable measurement data from which parameters can be analysed and then at processing parameters to provide information that can be used by a livestock farmer. The parameters are preferably recorded per hoof and can consist of values both for the front and the hind hooves and for left and right hooves. Parameters for the lateral and the medial halves of the hoof are recorded for each hoof and these parameters are sub-divided into caudial and cranial parameters for each half of the hoof. Using the software developed for this method various characteristics are calculated using the abovementioned recorded parameters. The characteristics of various abnormalities, such as foot rot, paralysis and Mortellaro's disease have been recorded. The measured characteristics are compared with the known recorded characteristics and the abnormalities are determined on the basis of this comparison.

The invention is differentiated from the human application in particular by the system that is necessary in order to obtain hoof prints of all four hooves, in an automated manner, that is to say without the need for human actions, to be able to identify the cow from which the prints originate, to be able to analyse which print originates from which hoof and to be able to analyse what step length is achieved. The spatial arrangement of the elements of one embodiment of the system 10 is shown in Fig. 2 and the system 10 is made up of the following components, which have already been described above with reference to Fig. 1;

Facilities 11 to control animal access to the measurement system; identification unit 12; sensor matrix (detector mat) 13 (optionally coupled to force recorders); presence detection sensors 14, 15; interface 17; and measurement computer 18 provided with analytical software.

The facilities 11 for controlling animal access to the measurement system, such as a gate that can be operated automatically, are used in combination with the presence detection sensors 14, 15 to ensure that measurement signals are always obtained for only one animal at a time. In order to obtain the correct measurement data it is important that the animals pass over the detector mat 13 without hindrance. The facilities 11 for controlling animal access to the measurement system are activated only if an animal approaches while the system 10 is still occupied by another animal or if an animal wants to approach the system 10 from the exit side. The new animal is held back until the system 10 is available again. The presence of animals is detected by presence detection sensors 14, 15. Preferably one or more proximity sensor(s) 16 to detect the approach of an animal are fitted in front of the entrance barrier and one or more presence detection sensors 14, 15 are fitted in the measurement section of the system 10 in order to check whether or not the measurement system is occupied. A presence detection sensor (not shown) can be fitted beyond the exit barrier in order to be able to detect the approach of an animal from the exit side of the system 10. These sensors can also be used to initiate and to stop the measurement process. The presence detection sensor 14 that is fitted after the entrance gate 11 and in front of the measurement system 13 emits a signal before the animal places its front feet on the measurement system 13. If the presence detection sensor that is fitted above the measurement system 13 emits a signal, the animal is standing with its front and/or hind feet on the measurement system 13. On the basis of the data from the measurement mat 13 and the signals from these two presence detection sensors 14, 15, the software determines which print belongs to which foot (front, hind, left, right).

An identification unit 12, by means of which the electronic identification number of the animal is read, is placed in the path between facilities 11 that are fitted to be able to bar access to the measurement system 13. If the farm does not use an electronic animal identification system, the identification unit can be replaced by a terminal which is used to enter the animal number. By identifying the animals it becomes possible to add data to a database and to generate alerts from the database by the farm management system 18. Without identification only online alerts can be generated.

The analysis on the measurement computer 18 is carried out in a number of steps. The first step in the analysis is the recognition of the hoof print patterns to be analysed. The measurements are carried out with a frequency of, for example, 200 measurements per second. During a measurement the entire sensor matrix of the measurement mat 13 is scanned. The measurement is activated as soon as the animal steps onto the measurement mat 13 and then remains active for a fixed time or for the time that the presence of one or more hooves on the measurement mat 13 is detected. On the basis of the data that become available during or after a measurement session, the analytical software determines which hoof print image or series of images is (are) suitable for processing. This can be, on the one hand, one specific image, or the average of a number of images where the hoof print is as complete as possible or, on the other hand, a series of images from which the changes in the hoof when it is put down and/or moved can be seen. Which animal and which hoof belong to a print is determined following this analysis. The animal number is read in by the identification unit (or terminal) 12. The hoof number is determined on the basis of the position of the print on the measurement mat 13, the position of the print with respect to the other hoof prints and the signals from the presence detection sensors 14, 15.

For each hoof, the hoof is subdivided into four segments. This subdivision can be made by, for example, determining the centre of gravity of the shape of the hoof print and then drawing a system of coordinates through this point, or by determining the position and direction of the cleft and the mid perpendicular of the cleft line. The point of engagement and the magnitude of the pressure that is exerted by the segment on the mat 13 are determined for the four segments.

Hoof abnormalities can be determined on the basis of:
differences in the pressure exerted by the different hooves on the mat;
differences in the pressure exerted by the segments of a hoof on the mat;
distance between the points of engagement of the segments of a hoof;
the width of the cleft;
the direction of the hoof with respect to the direction of movement; and/or
differences in the parameters from the current measurement compared with parameters from previous measurements (historical data).

The distance between the position of the hoof print of the front foot and the position of the hoof print of the hind foot (on one side of the animal) can also provide information with regard to the health of the hoof (the step length of the animal is indirectly measured with the aid of this method). In healthy animals the hind hoof is placed in or just behind the position where the front hoof was placed. In animals with hoof problems there can be some distance between the hoof prints (shorter step length).

## Claims

1. Method for monitoring animal hooves, comprising the steps of
detecting a pressure profile of at least one hoof of a cloven-hoofed animal;
deriving characteristic hoof parameters from the pressure profile; and
comparison of the detected hoof parameters with stored hoof parameters in order to detect a specific abnormality, **characterised in that**
the pressure profile is subdivided into four segments.

2. Method according to Claim 1, wherein the subdivision into the four segments is carried out using the centre of gravity of the pressure profile as the centre.

3. Method according to Claim 1, wherein the pressure profile is subdivided into four segments by detecting a cleft and subdividing the pressure profile along a direction parallel to the cleft and a direction perpendicular to the cleft.

4. Method according to one of the preceding claims, wherein the hoof parameters are determined for a lateral and a medial half of the hoof.

5. Method according to one of the preceding claims, wherein the hoof parameters comprise caudial and cranial parameters.

6. Method according to one of the preceding claims, wherein the hoof parameters comprise the width of a cleft.

7. Method according to one of the preceding claims, further comprising the step of identifying the cloven-hoofed animal.

8. Method according to one of the preceding claims, wherein the pressure profile is a dynamic pressure profile.

9. Method according to one of the preceding claims, wherein the pressure profile is a static pressure profile.

10. Method according to one of the preceding claims, wherein the stored hoof parameters are historically detected hoof parameters for the same animal.

11. Method according to one of the preceding claims, further comprising the step of identifying a hoof print as originating from a front, hind, left or right hoof of the animal.

12. Installation for monitoring animal hooves, comprising detection means (13) for the detection of a pressure profile of at least one hoof of a cloven-hoofed animal and processing means (18) which are connected to the detection means (13) and to memory means (19) for storing hoof parameters, wherein the processing means (18) are equipped to carry out the method according to one of Claims 1 to 11.

13. Installation according to Claim 12, further comprising identification means (12) which are connected to the processing means (18) for the identification of the cloven-hoofed animal.

14. Installation according to Claim 12 or 13, further comprising at least one presence detector (14, 15) which is connected to the processing means (18) for detecting the presence of the cloven-hoofed animal.

15. Installation according to Claim 12, 13, or 14, further comprising an access system that is connected to the processing means and controls access to the detection means (13).

## Patentansprüche

1. Verfahren zum Überwachen von Tierhufen mit den Schritten des Erkennens eines Druckprofils wenigstens eines Hufs eines paarzehigen Tiers; des Ableitens charakteristischer Hufparameter aus dem Druckprofil; und des Vergleichens der erkannten Hufparameter mit gespeicherten Hufparameter zur Erkennung einer bestimmten Abnormalität, **dadurch gekennzeichnet, daß** das Druckprofil in vier Segmente unterteilt ist.

2. Verfahren nach Anspruch 1, bei dem die Unterteilung in die vier Segmente unter Verwendung des Schwerpunkts des Druckprofils als Mittelpunkt erfolgt.

3. Verfahren nach Anspruch 1, bei dem das Druckprofil in vier Segmente unterteilt wird, indem eine Spalte erkannt wird und das Druckprofil entlang einer parallel zur Spalte verlaufenden Richtung und einer senkrecht zur Spalte verlaufenden Richtung unterteilt wird.

4. Verfahren nach einem der vorhergehenden Ansprüche, bei dem die Hufparameter für eine laterale und eine mediale Hälfte des Hufs ermittelt werden.

5. Verfahren nach einem der vorhergehenden Ansprüche, bei dem die Hufparameter kaudale und kraniale Parameter umfassen.

6. Verfahren nach einem der vorhergehenden Ansprüche, bei dem die Hufparameter die Breite einer Spalte umfassen.

7. Verfahren nach einem der vorhergehenden Ansprüche, ferner mit dem Schritt des Identifizierens des paarzehigen Tiers.

8. Verfahren nach einem der vorhergehenden Ansprüche, bei dem das Druckprofil ein dynamisches Druckprofil ist.

9. Verfahren nach einem der vorhergehenden Ansprüche, bei dem das Druckprofil ein statisches Druckprofil ist.

10. Verfahren nach einem der vorhergehenden Ansprüche, bei dem die gespeicherten Hufparameter in der Vergangenheit erkannte Hufparameter des selben Tiers sind.

11. Verfahren nach einem der vorhergehenden Ansprüche, ferner mit dem Schritt des Identifizierens eines Hufabdrucks als von einem vorderen, hinteren, linken oder rechten Huf des Tieres stammend.

12. Vorrichtung zum Überwachen von Tierhufen mit einer Erkennungseinrichtung (13) zum Erkennen eines Druckprofils wenigstens eines Hufs eines paarzehigen Tiers und einer Verarbeitungseinrichtung (18), die mit der Erkennungseinrichtung (13) und einer Speichereinrichtung (19) zum Speichern der Hufparameter verbunden ist, wobei die Verarbeitungseinrichtung (18) zur Durchführung des Verfahrens nach einem der Ansprüche 1 bis 11 ausgebildet ist.

13. Vorrichtung nach Anspruch 12, ferner mit einer Identifizierungseinrichtung (12), die mit der Verarbeitungseinrichtung (18) zum Identifizieren des paarzehigen Tiers verbunden ist.

14. Vorrichtung nach Anspruch 12 oder 13, ferner mit wenigstens einem Anwesenheitsdetektor (14, 15), der mit der Verarbeitungseinrichtung (18) zum Erkennen der Anwesenheit eines paarzehigen Tiers verbunden ist.

15. Vorrichtung nach Anspruch 12, 13 oder 14, ferner mit einem Zugangssystem, das mit der Verarbeitungseinrichtung verbunden ist und den Zugang zur Erkennungseinrichtung (13) steuert.

## Revendications

1. Procédé de surveillance de sabots d'animaux, comprenant les étapes consistant à détecter un profil de pression d'au moins un sabot d'un animal à sabots fendus, établir des paramètres de sabot caractéristiques à partir du profil de pression, et comparer les paramètres de sabot détectés avec des paramètres de sabot mémorisés afin de détecter une anomalie spécifique, **caractérisé en ce que** le profil de pression est subdivisé en quatre segments.

2. Procédé selon la revendication 1, dans lequel la subdivision en quatre segments est exécutée en utilisant le centre de gravité du profil de pression comme centre.

3. Procédé selon la revendication 1, dans lequel le profil de pression est subdivisé en quatre segments en détectant une fissure et en subdivisant le profil de pression suivant une direction parallèle à la fissure et une direction perpendiculaire à la fissure.

4. Procédé selon l'une des revendications précédentes, dans lequel les paramètres de sabot sont déterminés pour une moitié latérale et une moitié interne du sabot.

5. Procédé selon l'une des revendications précédentes, dans lequel les paramètres de sabot comprennent des paramètres postérieurs et antérieurs.

6. Procédé selon l'une des revendications précédentes, dans lequel les paramètres de sabot comprennent la largeur d'une fissure.

7. Procédé selon l'une des revendications précédentes, comprenant en outre l'étape consistant à identifier l'animal à sabots fendus.

8. Procédé selon l'une des revendications précédentes, dans lequel le profil de pression est un profil de pression dynamique.

9. Procédé selon l'une des revendications précédentes, dans lequel le profil de pression est un profil de pression statique.

10. Procédé selon l'une des revendications précédentes, dans lequel les paramètres de sabot mémorisés sont des paramètres de sabot détectés de façon chronologique pour le même animal.

11. Procédé selon l'une des revendications précédentes, comprenant en outre l'étape consistant à identifier une empreinte de sabot comme provenant d'un sabot avant, arrière, gauche ou droit de l'animal.

12. Installation permettant de surveiller des sabots d'animaux, comprenant un moyen de détection (13) servant à la détection d'un profil de pression d'au moins un sabot d'un animal à sabots fendus et de moyens de traitement (18) qui sont reliés au moyen de détection (13) et à un moyen de mémoire (19) servant à mémoriser des paramètres de sabot, dans laquelle les moyens de traitement (18) sont équipés pour exécuter le procédé de l'une des revendications 1 à 11.

13. Installation selon la revendication 12, comprenant en outre des moyens d'identification (12) qui sont reliés aux moyens de traitement (18) pour l'identification de l'aninal à sabots fendus.

14. Installation selon la revendication 12 ou 13, comprenant en outre au moins un détecteur de présence (14, 15) qui est relié aux moyens de traitement (18) pour détecter la présence de l'animal à sabots fendus.

15. Installation selon la revendication 12, 13 ou 14, comprenant en outre un système d'accès qui est connecté aux moyens de traitement et contrôle l'accès au moyen de détection (13).
